(19) Europäisches Patentamt
European Patent Office
Office européen des brevets

(11) Veröffentlichungsnummer : **0 037 482**
**B1**

# (12) EUROPÄISCHE PATENTSCHRIFT

(45) Veröffentlichungstag der Patentschrift : **13.03.85**

(51) Int. Cl.[4] : **C 07 D285/00**, A 01 N 43/72

(21) Anmeldenummer : **81101929.8**

(22) Anmeldetag : **16.03.81**

(54) **3,4,5,6-Tetrahydro-1,2,4,6-thiatriazin(3,5)dion-1,1-dioxide, ihre Herstellung, sie enthaltende Herbizide, Verfahren zur Bekämpfung unerwünschten Pflanzenwachstums und zur Herstellung von Herbiziden.**

(30) Priorität : **05.04.80 DE 3013268**

(43) Veröffentlichungstag der Anmeldung : **14.10.81 Patentblatt 81/41**

(45) Bekanntmachung des Hinweises auf die Patenterteilung : **13.03.85 Patentblatt 85/11**

(84) Benannte Vertragsstaaten : **AT BE CH DE FR GB IT LI NL SE**

(56) Entgegenhaltungen :
**US-A- 3 435 031**
**US-A- 3 817 993**
**US-A- 3 856 786**
**Chemie der Pflanzenschutz- und Schädlingsbekämpfungsmittel, Band 2 (R. Wenler), Springer, 1970, S. 359**
**Römpps Chemie Lexikon, 8. Auflage, S. 112**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Acker, Rolf-Dieter, Dr.**
**Tuchbleiche 8**
**D-6906 Leimen (DE)**
Erfinder : **Hamprecht, Gerhard, Dr.**
**Rote-Turm-Strasse 28**
**D-6940 Weinheim (DE)**
Erfinder : **Parg, Adolf, Dr.**
**Paray-Le-Monial-Strasse 8**
**D-6702 Bad Duerkheim (DE)**
Erfinder : **Wuerzer, Bruno, Dr.**
**Ruedigerstrasse 13**
**D-6701 Otterstadt (DE)**

**EP 0 037 482 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue 3,4,5,6-Tetrahydro-1,2,4,6-thiatriazin(3,5)dion-1,1-dioxide, Verfahren zu ihrer Herstellung, Herbizide, die diese Verbindungen als Wirkstoffe enthalten, sowie Verfahren zur Bekämpfung unerwünschten Pflanzenwuchses mit diesen Verbindungen.

In der US-PS 3 435 031 werden substituierte 3,4,5,6-Tetrahydro-1,2,4,6-thiatriazin(3,5)dion-1,1-dioxide der Formel

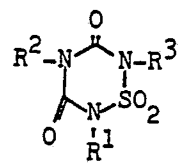

beschrieben, worin $R^1$ und $R^2$ gleich oder verschieden sein können und u. a. einen $C_{1-12}$-Alkylrest, einen $C_{3-6}$-Cycloalkylrest oder einen Phenyl-($C_{1-3}$-alkyl)-rest und $R^3$ u. a. einen $C_{1-3}$-Alkyrest oder einen $C_{1-3}$-Alkanoylmethylrest bedeuten können. Diese Verbindungen sollen sich als Bakterizide, Fungizide und Fliegen-Repellentien eignen. Eine herbizide Wirkung wurde bisher für diese Verbindungen nicht bekannt.

Gemäß US-PS 3 817 993 besitzen 2,4,6-substituierte 3,5-Dioxo-1,2,4,6-thiatriazine eine herbizide Wirkung. Diese Substanzen wirken jedoch erst in sehr hohen Konzentrationen.

Es wurden nun Thiatriazin-Derivate gefunden, die eine herbizide Wirkung besitzen. Gegenstand der Erfindung sind 3,4,5,6-Tetrahydro-thiatriazin(3,5)dion-1,1-dioxide der Formel I

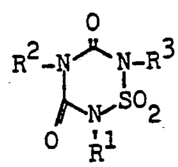

(I)

worin

$R^1$ Wasserstoff, ein Metallatom, einen gegebenenfalls $C_{1-3}$-alkylsubstituierten Ammoniumrest, einen gesättigten geradkettigen aliphatischen Rest mit bis zu 10 Kohlenstoffatomen, einen cycloaliphatischen Rest mit 3 bis 7 Kohlenstoffatomen, einen verzweigten gesättigten aliphatischen Rest mit 3 bis 10 Kohlenstoffatomen, oder einen gegebenenfalls halogensubstituierten Benzylrest,

$R^2$ Wasserstoff, ein Metallatom, einen gesättigten aliphatischen Rest mit bis zu 10 Kohlenstoffatomen,

$R^3$ einen halogen-, alkyl-, halogenalkylthio-, halogenalkylsulfonyl-substituierten Phenylrest mit bis zu 10 Kohlenstoffatomen bedeuten.

$R^1$ und $R^2$ bedeuten beispielsweise Natrium, Kalium, Ammonium, Dimethylammonium, Trimethylammonium, Diisopropylethylammonium, Diisopropylmethylammonium.

$R^2$ bedeutet beispielsweise Methyl, Ethyl, Propyl, Isopropyl, Cyclopropyl, Butyl, Isobutyl, sec.-Butyl, tert.-Butyl, Pentyl, Cyclopentyl, Hexyl, Cyclohexyl, 3-Pentyl, 1,2-Dimethylpropyl, 1,3-Dimethylbutyl, 1-Ethyl-2-methylpropyl, 1,2,2-Trimethylpropyl, 1,2-Dimethylhexyl, 1-Cyclohexylethyl, tert.-Amyl.

Die Bezeichnung « Halogen » bedeutet Fluor, Chlor Brom oder Jod.

$R^3$ kann beispielsweise o-, m-, p-Chlorphenyl, o-, m-, p-Fluorphenyl, o-, m-, p-Bromphenyl, 2,3-, 2,4-, 2,5-, 2,6-, 3,4-, 3,5-Dichlorphenyl, 2,4,5-Trichlorphenyl, 3,4-Difluorphenyl, 3-Chlor-4-fluorphenyl, 3-Fluor-4-chlorphenyl, o-, m-, p-Methyl-, o-, m-, p-Ethyl, o-, m-, p-Propyl, o-, m-, p-Isopropyl-, o-, m-, p-Isobutyl-, o-, m-, p-tert.-Butyl-, o-, m-, p-Trichlormethylthio-, o-, m-, p-Trifluormethylthio-, o-, m-, p-Trifluormethylsulfonyl, 3-Chlor-4-brom-, 3-Chlor-4-methyl-phenyl bedeuten.

Die neuen Verbindungen der Formel I lassen sich herstellen, indem man

a) — falls $R^1$ Wasserstoff bedeutet — einen N-Aryl-N'-Alkyl-Harnstoff der Formel II

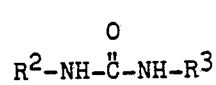

(II)

in der $R^2$ und $R^3$ die vorgenannten Bedeutungen besitzen, mit Chlorsulfonylisocyanat, gegebenenfalls in Gegenwart eines Säureakzeptors und eines inerten Lösungsmittels bei einer Temperatur im Bereich von − 40 bis 100 °C oder

b) — falls $R^2$ Wasserstoff bedeutet — ein Sulfondiamid der Formel III

$$R^1—NH—SO_2—NH—R^3 \qquad (III)$$

in der $R^1$ und $R^3$ die vorgenannten Bedeutungen besitzen, mit Chlorcarbonylisocyanat, gegebenenfalls in Gegenwart eines Säureakzeptors und eines inerten organischen Lösungsmittels bei einer Temperatur im Bereich von 0 °C bis 150 °C oder

c) einen Harnstoff der Formel II

$$R^2\text{-NH-}\overset{O}{\overset{\|}{C}}\text{-NH-}R^3 \qquad (II)$$

oder einen Harnstoff der Formel IV

$$R^2—NH—CO—NH_2 \qquad (IV)$$

wobei $R^2$ und $R^3$ die vorgenannte Bedeutung besitzen, mit einem Sulfamoylchlorid der Formel V

$$\begin{array}{l} R^1\text{-N-SO}_2\text{Cl} \\ \quad\;\text{COCl} \end{array} \qquad (V)$$

oder einem Sulfamoylchlorid der Formel VI

$$\begin{array}{l} R^1\text{-N-SO}_2\text{-Cl} \\ \quad\;\text{CO}_2R^4 \end{array} \qquad (VI)$$

in der $R^1$ die vorgenannte Bedeutung, ausgenommen Wasserstoff, ein Metallatom oder einen gegebenenfalls $C_{1-3}$-alkylsubstituierten Ammoniumrest besitzt, und $R^4$ Alkyl oder Cycloalkyl mit bis zu 6 Kohlenstoffatomen bedeutet, gegebenenfalls in Gegenwart eines Säureakzeptors und eines inerten Lösungsmittels bei einer Temperatur von – 20 bis 100 °C oder

d) ein Sulfondiamid der Formel III mit einem Carbamoylchlorid der Formel VII

$$\begin{array}{l} R^2\text{-N-CO-O}R^4 \\ \quad\;\text{CO-Cl} \end{array} \qquad (VII)$$

oder einem Carbamoylchlorid der Formel VIII

$$\begin{array}{l} R^2\text{-N-CO-Cl} \\ \quad\;\text{CO-Cl} \end{array} \qquad (VIII)$$

in der $R^2$ und $R^4$ die vorgenannte Bedeutung, ausgenommen Wasserstoff oder Metallatome besitzen, gegebenenfalls in Gegenwart eines Säureakzeptors und eines inerten Lösungsmittels bei einer Temperatur von – 20 °C bis 100 °C oder

e) — falls $R^2$ Wasserstoff bedeutet — ein Sulfamoylchlorid der Formel IX

$$\begin{array}{l} R^1\text{-N-SO}_2\text{Cl} \\ \quad\;\text{CO-NCO} \end{array} \qquad (IX)$$

in der $R^1$ die vorgenannte Bedeutung besitzt, ausgenommen Wasserstoff, Metallatome und gegebenenfalls $C_{1-3}$-alkylsubstituierte Ammoniumreste, mit einem Amin der Formel X

$$R^3—NH_2 \qquad (X)$$

in der $R^3$ die vorgenannte Bedeutung besitzt, gegebenenfalls in Gegenwart eines Säureakzeptors und eines inerten Lösungsmittels bei einer Temperatur von – 100 °C bis 100 °C reagieren läßt und die so erhaltenen Verbindungen gegebenenfalls in ihre Salze überführt und/oder — falls $R^1$ Wasserstoff

bedeutet — alkyliert.

Das Verfahren a) läßt sich durch folgendes Formelschema wiedergeben :

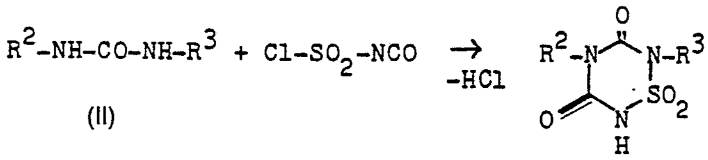


(II) (I)

Die Ausgangsstoffe werden in ungefähr stöchiometrischem Verhältnis eingesetzt, d. h. in einem Unter- bzw. Überschuß von bis zu 20 % Chlorsulfonylisocyanat bezogen auf II. Gegebenenfalls kann ein Säureakzeptor zur Vervollständigung der Reaktion zugesetzt werden.

Überraschend ist der regiospezifisch einheitliche Reaktionsablauf. Trotz der hohen Reaktivität des Chlorsulfonylisocyanats, der Anwesenheit zweier Amidstickstoffe und der bekannten Sulfonierung von Anilinen (J. chem. Soc. Perkin I, 1043 (1979)) trat unter den aufgezeigten Reaktionsbedingungen weder eine Sulfonierung des zweiten Stickstoffatoms noch des Arylkerns auf.

Zweckmäßig wird das Verfahren so durchgeführt, daß man das Chlorsulfonylisocyanat bei − 40 °C bis 100 °C, vorzugsweise − 20 °C bis 40 °C zu dem Harnstoff der Formel II und einem inerten Lösungsmittel zulaufen läßt. Gegebenenfalls kann der Säureakzeptor nach 0,5 bis 48 Stunden, vorzugsweise 1 bis 12 Stunden bei einer Temperatur von − 20 °C bis 40 °C in Mengen von 0,5 bis 1,5 Äquivalenten zugegeben werden. Zur Beendigung der Umsetzung rührt man 0,5 bis 48 Stunden, vorzugsweise 2 bis 12 Stunden bei − 20 °C bis 80 °C nach. Das Reaktionsgemisch wird eingeengt. Die gewünschten Endstoffe der Formel I können durch Umfällen, Umkristallisieren oder Ausfällen als Salze rein isoliert werden, gegebenenfalls können sie durch Chromatographie gereinigt werden.

Das Verfahren b) läßt sich durch folgendes Formelschema wiedergeben :

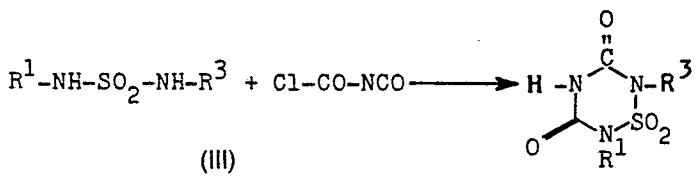

(III) (I)

Die Ausgangsstoffe werden in ungefähr stöchiometrischem Verhältnis eingesetzt, d. h. in einem Unter- bzw. Überschuß von bis zu 20 % Chlorcarbonylisocyanat, bezogen auf III. Gegebenenfalls kann ein Säureakzeptor zur Vervollständigung der Reaktion zugesetzt werden.

Überraschend ist die glatte Bildung eines Triazinonsystems auf intramolekularem Wege aus Chlorcarbonylisocyanat und Sulfondiamiden, da bei dem reaktiven Chlorcarbonylisocyanat erwartet werden kann, daß intramolekulare Verknüpfungen zwischen beiden trifunktionellen Systemen überwiegen. In diesem Zusammenhang ist die Bildung eines regiospezifischen Produkts besonders überraschend.

Zweckmäßig wird das Verfahren so durchgeführt, daß man das Chlorcarbonylisocyanat bei 0 bis 150 °C, vorzugsweise bei 10 °C bis 50 °C zu dem Sulfondiamid III und einem inerten Lösungsmittel zulaufen läßt. Gegebenenfalls kann der Säureakzeptor nach 0,5 bis 24 Stunden, vorzugsweise 1 bis 12 Stunden bei einer Temperatur von 0 °C bis 40 °C in Mengen von 0,5 bis 1,5 Äquivalenten zugegeben werden. Zur Beendigung der Umsetzung rührt man 1 bis 48 Stunden, vorzugsweise 2 bis 12 Stunden bei 80 °C bis 130 °C nach. Die gewünschten Endstoffe der Formel I können nach dem Filtrieren gegebenenfalls durch Ausfällen oder Einengen des Reaktionsgemisches erhalten werden, gegebenenfalls können sie durch Umkristallisieren oder Chromatographie gereinigt werden.

Das Verfahren c) läßt sich durch folgende Formelschemen wiedergeben :

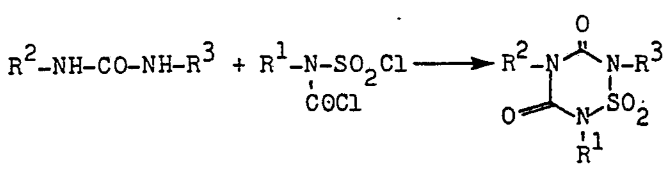

(II) (V) (I)

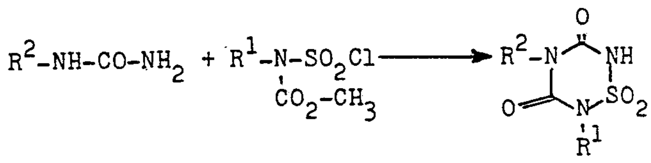

(IV) (VI) (I)

Die Ausgangsstoffe II und V bzw. IV und VI werden in stöchiometrischem Verhältnis eingesetzt, d. h. in einem Unter- oder Überschuß von bis zu 20 % Ausgangsstoff II und IV, bezogen auf V bzw. VI.

Zweckmäßig wird das Verfahren so durchgeführt, daß man über zwei Zuführungen das Carbamoylchlorid V bzw. VI und die äquivalente Menge an Säureakzeptor bei einer Temperatur von – 20 °C bis 100 °C, vorzugsweise bei 0 bis 40 °C zu einer ungefähr äquivalenten Menge an Harnstoff der Formel IV in einem inerten Lösungsmittel zulaufen läßt. Man rührt anschließend noch 2 bis 24 Stunden bei 0 bis 100 °C, vorzugsweise 20 bis 60 °C nach.

Bei Verwendung von Sulfamoylchloriden der Formel V wird gegebenenfalls eingeengt, bzw. im Falle mit Wasser nicht mischbarer Lösungsmittel mit verd. Salzsäure zur Entfernung der Hydrochloride extrahiert. Die gewünschten Endstoffe der Formel I können dann gegebenenfalls durch Umkristallisieren oder Chromatographie gereinigt werden.

Bei Verwendung von Sulfamoylchloriden der Formel VI kann die Cyclisierung gegebenenfalls nach dem Abtrennen der Hydrochloride in Gegenwart der 1- bis 2,5-fachen Menge Base oder auch im organischen Medium in Gegenwart der 1 bis 2,5-fachen Menge Alkoholat bei einer Temperatur von – 20 °C bis 100 °C, vorzugsweise 20 °C bis 80 °C erfolgen. Zur Aufarbeitung der Endstoffe der Formel I säuert man danach an und saugt den angefallenen Niederschlag, gegebenenfalls nach weiterem Einengen, ab. Die gewünschten Endstoffe fallen hierbei in reiner Form an, gegebenenfalls können sie durch Umkristallisieren oder Chromatographie gereinigt werden.

Das Verfahren d) verläuft nach folgenden Schemen :

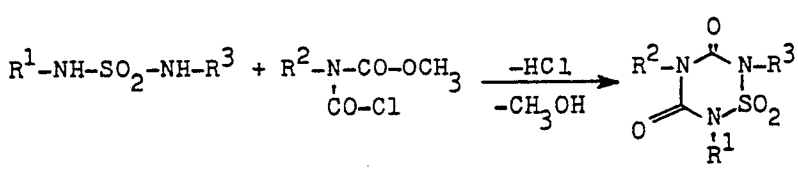


(III) (VII) (I)

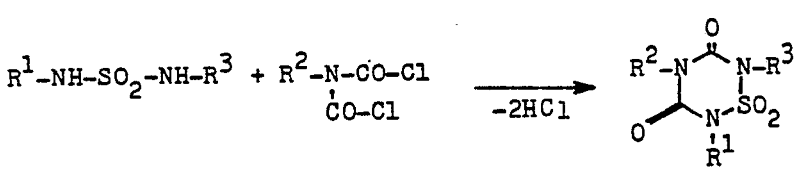


(III) (VIII) (I)

Die Ausgangsstoffe III und VII bzw. VIII werden in stöchiometrischem Verhältnis eingesetzt, d. h. in einem Unter- oder Überschuß von bis zu 20 % Ausgangsstoff III bezogen auf VII bzw. VIII.

Zweckmäßig wird das Verfahren so durchgeführt, daß man über zwei Zuführungen das Carbamoylchlorid VII bzw. VIII und die äquivalente Menge an Säureakzeptor bei einer Temperatur von 20 °C bis 100 °C, vorzugsweise bei 0 bis 40 °C zu einer ungefähr äquivalenten Menge an Sulfondiamid der Formel III in einem inerten Lösungsmittel zulaufen läßt. Zur Beendigung der Umsetzung rührt man noch 2 bis 24 Stunden bei 0 bis 100 °C, vorzugsweise 20 bis 60 °C nach.

Bei Verwendung von Carbamoylchloriden der Formel VIII wird das Reaktionsgemisch dann gegebenenfalls eingeengt, bzw. im Falle mit Wasser nicht mischbarer Lösungsmittel mit verd. Salzsäure zur Entfernung der Hydrochloride extrahiert. Die gewünschten Endstoffe der Formel I können dann gegebenenfalls durch Umkristallisieren oder Chromatographie gereinigt werden.

Bei Verwendung von Carbamoylchloriden der Formel VII kann die Cyclisierung gegebenenfalls nach

dem Abtrennen der Hydrochloride in Gegenwart der 1- bis 2,5-fachen Menge Base oder auch im organischen Medium in Gegenwart der 1- bis 2,5-fachen Menge Alkoholat bei einer Temperatur von – 20 °C bis 100 °C, vorzugsweise 20 °C bis 80 °C erfolgen. Zur Aufarbeitung der Endstoffe der Formel I säuert man danach an und saugt den angefallenen Niederschlag, gegebenenfalls nach weiterem Einengen, ab. Die gewünschten Endstoffe fallen hierbei in reiner Form an, gegebenenfalls können sie durch Umkristallisieren oder Chromatographie gereinigt werden.

Die Reaktion e) läuft wie folgt :

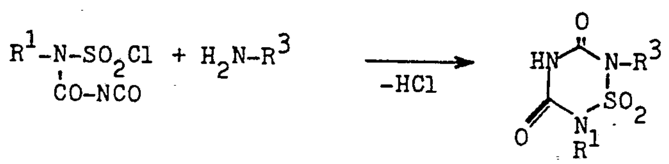

(IX) (X) (I)

Die Ausgangsstoffe IX und X werden in stöchiometrischen Verhältnissen eingesetzt, d. h. in einem Unter- oder Übershuß von bis zu 20 % Ausgangsstoff IX, bezogen auf X.

Zweckmäßig wird das Verfahren so durchgeführt, daß man das Amin X gegebenenfalls in einem inerten Lösungsmittel zu einer Lösung des Sulfamoylchlorids IX in einem inerten Lösungsmittel bei einer Temperatur von – 100 °C bis 50 °C, vorzugsweise – 80 °C bis 0 °C zulaufen läßt. Zum vollständigen Ablauf der Reaktion kann man 0,5 bis 24 Stunden, vorzugsweise 1 bis 12 Stunden bei einer Temperatur von 0 ° bis 100 °C, vorzugsweise 20 °C bis 80 °C nachrühren lassen.

Zur Isolierung der Endstoffe der Formel I wird der Niederschlag abgesaugt oder die Reaktionsmischung gegebenenfalls eingeengt. Man kann dann durch Zugabe der 1 bis 2,5-fachen Menge einer Base und anschließenden Ansäuern das gewünschte Thiatriazin I rein erhalten, gegebenenfalls kann eine Reinigung auch durch Umkristallisieren oder Chromatographie erfolgen.

Die anschließende Alkylierung so erhaltener Verbindungen, in denen $R^1$ und/oder $R^2$ Wasserstoff bedeutet, kann mit den entsprechenden Alkylhalogeniden oder mit Dialkylsulfaten erfolgen. Als solche kommen vorzugsweise Methyliodid und Dimethylsulfat in Betracht.

Zweckmäßig wird die Alkylierung so durchgeführt, daß man das Alkylierungsmittel zur Verbindung der Formel I ($R^1$ = H oder $R^2$ = H) und gegebenenfalls einem Säureakzeptor in einem inerten Lösungsmittel bei einer Temperatur von 0° bis 100 °C, vorzugsweise 10 °C bis 40 °C zulaufen läßt. Man rührt anschließend noch 2 bis 24 Stunden bei 0 °C bis 100 °C, vorzugsweise bei 20 °C bis 60 °C.

Die Endstoffe der Formel I können gegebenenfalls durch Ausfällen, Einengen der Lösung und Umkristallisieren bzw. durch Chromatographie rein isoliert werden.

Die erfindungsgemäßen Verfahren können kontinuierlich oder diskontinuierlich, drucklos oder unter Druck durchgeführt werden, der Einfachheit halber wird bei den Verfahren Atmosphärendruck bevorzugt.

Man verwendet für die erfindungsgemäßen Verfahren unter den jeweiligen Reaktionsbedingungen inerte organische Lösungsmittel. Als Lösungsmittel kommen z. B. in Frage : Halogenkohlenwasserstoffe, insbesondere Chlorkohlenwasserstoffe, z. B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-,m-mp-Difluorbenzol, 1,2-Dichlorethan, 1,1-Dichlorethan, 1,2-cis-Dichlorethylen, Chlorbenzol, Fluorbenzol, Brombenzol, Jodbenzol, o-, p- und m-Dichlorbenzol, o-, p-, m-Dibrombenzol, o-, m-, p-Chlortoluol, -1,2,4-Trichlorbenzol ; Ether, z. B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thionisol, β, β'-Dichlordiethylether ; Nitrokohlenwasserstoffe wie Nitromethan, Nitroethan, Nitrobenzol, o-, m-, p-Chlornitrobenzol, o-Nitrotoluol ; aliphatische oder cycloaliphatische Kohlenwasserstoffe, z. B. Heptan, Pinan, Nonan, o-, m-, p-Cymol, Benzinfraktionen innerhalb eines Siedepunktintervalls von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan ; Ester, z. B. Ethylacetat, Acetessigester, Isobutylacetat ; Amide, z. B. Formamid, Methylformamid, Dimethylformamid ; Ketone, z. B. Aceton, Methylethylketon ; und enstprechende Gemische. Zweckmäßig verwendet man das Lösungsmittel in einer Menge von 100 bis 2 000 Gew.%, vorzugsweise 200 bis 700 Gew.%, bezogen auf die Ausgangsstoffe.

Eine anschließende Alkylierung wird zweckmäßig in Wasser oder Alkoholen, wie z. B. Methanol, Ethanol, n-Propanol, i-Propanol und Butanol durchgeführt.

Als Säureakzeptoren können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Es kommen z. B. basische Verbindungen in Frage : Kaliumhydroxid, Natriumhydroxid, Kaliumcarbonat,

Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tripropylamin, Triisopropylamin, Tributylamin, Triisobutylamin, Tri-sec-butylamin, Tri-tert.butylamin, Tribenzylamin, Tricyclohexylamin, Triamylamin, Diisopropylethylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyrridin, N,N-Diethyl-p-aminopyridin, N,N-Dipropyl-p-aminopyridin, N-Methylpyrrolidon, N-Ethylpyrrolidon, N-Methylpiperidin, N-Ethylpiperidin, N-Methylpyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethyleninim, N-Ethylhexamethylenimin, Pyridin, Chinolin, α-Picolin, β-Picolin, γ-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N'-Dimethylcyclohexylamin, 2,6-Lutidin, 2,4-Lutidin, Trifurylamin, Triethylendiamin.

Bei Verwendung der Einsatzstoffe der Formeln VI und VII können gegebenenfalls Stoffe zugesetzt werden, die eine Ringschlußreaktion beschleunigen oder in Gang setzen.

Als Cyclisierungsagentien kommen z. B. die vorgenannten anorganischen Säurebindemittel in Frage, ferner z. B. Natriumpropionat, Natriumbutyrat, Natriumisobutyrat, Kaliumformiat, Kaliumacetat, Kaliumpropionat, Kaliumbutyrat, Kaliumisobutyrat, Natriummethylat, Natriumethylat, Natriumpropylat, Natriumisopropylat, Natriumbutylat, Natriumsobutylat, Natrium-sec-butylat, Natrium-tert-butylat, Natriumethylenglykolat, Natriumpropylen-(1,2)-glykolat, Natriumpropylen-(1,3)-glykolat, Natriumdiethylenglykolat, Natriumtriethylenglykolat, Natriumdipropylen-(1,2)-glykolat, Kaliummethylat, Kaliumethylat, Kalium-n-propylat, Kaliumisopropylat, Kalium-n-butylat, Kalium-isobutylat, Kalium-sec-butylat, Kaliumtert-butylat, Kaliumethylenglykolat, Kaliumpropylen-(1,2)-glykolat, Kaliumpropylen-(1,3)-glykolat, Kaliumdiethylenglykolat, Kaliumtriethylenglykolat, Kaliumdipropylen-(1,2)-glykolat.

Das Säurebindemittel wird zweckmäßig in äquivalenten Mengen oder in einem Überschuß von bis zu 20 % bezogen auf die Ausgangsstoffe VI oder VII eingesetzt.

Die Ausgangsmaterialien der genannten Verfahren sind bekannt oder lassen sich nach bekannten Methoden herstellen. Ausgangsstoffe der Formel V und VI sind aus der DE-OS 28 28 969 bekannt, Ausgangsstoff VII ist in der DE-AS 12 59 871 beschrieben. Das Carbamoylchlorid VIII findet Anwendung in der DE-OS 26 50 014.

## Beispiel 1

65,7 Teile N-Methyl-N'(3',4'-Dichlor-phenyl)-harnstoff werden in 300 Teilen Dioxan suspendiert. Man fügt bei Raumtemperatur portionsweise 42,5 Teile Chlorsulfonylisocyanat zu. Nach 16 Stunden Rühren wird vom Ungelösten filtriert und die Lösung eingeengt ; Lösungsmittelreste werden bei 40 bis 60 °C/0,01 mbar entfernt. Zum Rückstand gibt man 90 Teile Essigester und setzt nach dem Kristallisieren 200 ml Petrolether zu. Nach dem Absaugen und Trocknen erhält man 75 Teile 2H-4-Methyl-6-(3',4'-dichlorphenyl)-3,4,5,6-tetrahydro-1,2,3,6-thiatriazin(3,5)dion-1,1-dioxid (Wirkstoff 1), Schmp. 180 bis 183 °C.

## Beispiel 2

15,4 Teile Chlorsulfonylisocyanat werden zu einer Suspension von 20 Teilen N-Methyl-N'-(4'-chlorphenyl)-harnstoff in 130 Teilen Dioxan portionsweise bei 35 bis 40 °C zugefügt. Man rührt 8 Stunden, fügt 12 Teile Triethylamin zu und rührt nochmals 12 Stunden nach. Nach dem Filtrieren wird die Lösung eingeengt. 15 Teile einer 30 %igen Natriummethylatlösung werden hinzugegeben ; das Methanol wird anschließend abdestilliert. Lösungsmittelreste lassen sich bei 50 bis 60 °C/0,1 mbar entfernen. Man nimmt in Methylenchlorid auf, rührt 1 bis 2 Stunden und saugt den Niederschlag ab. Man erhält 19 Teile 2-Natrium-4-methyl-6-(4'-chlorphenyl)-3,4,5,6-tetrahydro-1,2,4,6-thiatriazin(3,5)-dion-1,1-dioxid (Wirkstoff 2), Schmp. > 240 °C.

## Beispiel 3

43,8 Teile N-Methyl-N'-(3', 4',-dichlorphenyl)-harnstoff werden in 180 Teilen Dioxan vorgelegt. 28,3 Teile Chlorsulfonylisocyanat werden bei 12-15 °C portionsweise zugefügt, man läßt 12 Stunden bei Raumtemperatur nachrühren.

Man läßt 20,2 Teile Triethylamin zwischen 10° und 12 °C zulaufen, anschließend wird 30 Stunden bei Raumtemperatur gerührt. Nach dem Abfiltrieren vom Ungelösten wird das Filtrat im Vakuum eingeengt. 18 Teile 0,1 molarer Natriummethylat-Lösung in Methanol werden 0° bis 5° zugegeben. Nach 30 Minuten Rühren wird der Niederschlag abfiltriert und getrocknet. Man erhält 42,1 Teile 2H-4-Methyl-6-(3',4'-dichlorphenyl)-3,4,5,6-tetrahydro-1,2,4,6-thiatriazin-(3,5)-dion-1,1-dioxid, das mit zirka 0,5 Mol Dioxan kristallisiert, Schmp. > 240 °C (Wirkstoff 3).

## Beispiel 4

10 Teile des Wirkstoffs 1 werden in 100 ml trockenem Ethanol vorgelegt, 10,5 Teile einer 2,9 molaren Natriummethylatlösung in Ethanol werden zugefügt und 15 Minuten bei Raumtemperatur nachgerührt. Abdestillieren des Lösungsmittels und Umfällen aus Essigester/Petrolether ergibt 10,3 Teile Natriumsalz des 4-Methyl-6-(3',4'-dichlorphenyl)-3,4,5,6-tetrahydro-1,2,4,6-thiatraizin-(3,5)-dion-1,1-dioxids (Wirkstoff 4), Schmp. > 240 °C.

Beispiel 5

22 Teile N-Methyl-N'-(4'-chlorphenyl)-sulfondiamid werden in 180 Teilen Toluol vorgelegt. 12,7 Teile Chlorcarbonylisocyanat werden bei 20 bis 25 °C portionsweise zugefügt. Nach 1 Stunde Rühren bei Raumtemperatur wird 6 Stunden unter Rückfluß erhitzt. Nach dem Filtrieren wird eingeengt und das Produkt mit Toluol/Petrolether ausgefüllt. Der nach dem Absaugen in Isopropanol angerührte Rückstand enthält 15,0 Teile reines 2-Methyl-4H-6-(4'-chlorphenyl)-3,4,5,6-tetrahydro-1,2,4,6-thiatriazin(3,5)dion-1,1-dioxid (Wirkstoff 5), Schmp. 170 bis 172 °C.

Beispiel 6

Zu 25,6 Teilen N-Isopropyl-N'-(4-chlorphenyl)-sulfondiamid und 18,0 Teilen Toluol werden bei Raumtemperatur 12,7 Teile Chlorcarbonylisocyanat portionsweise zugefügt. Nach 1 Stunde Rühren bei Raumtemperatur und 4 Stunden unter Rückfluß wird das Produkt nach dem Filtrieren mit Pentan ausgefällt. Man erhält 26 Teile 2-Isopropyl-4H-(4'-chlorphenyl)-3,4,5,6-tetrahydro-1,2,4,6-thiatriazin(3,5)dion-1,1-dioxid (Wirkstoff 6), Schmp. 130 bis 133 °C (Wirstoff 6).

Beispiel 7

8,0 Teile des Wirstoffes 5 (Beispiel 6), 1,1 Teile Natriumhydroxid, 30 Teile Wasser und 25 Teile Methanol werden zusammengegeben. Dann fügt man portionsweise 7,7 Teile Dimethylsulfat bei 25 bis 30 °C zu und rührt 3 Stunden bei Raumtemperatur nach. Nach Zugabe von 18 Teilen konzentrierter wäßriger Ammoniaklösung wird abgesaugt und der Rückstand getrocknet. Es ergeben sich 7,8 Teile 2,4-Dimethyl-6-(4'-chlorphenyl)-3,4,5,6-tetrahydro-1,2,4,6-thiatriazin(3,5)dion-1,1-dioxid (Wirkstoff 7), Schmp. 82 bis 84 °C.

Analog Beispiel 1 bis 7 wurden hergestellt :

(Siehe Tabelle, Seite 9 ff.)

Tabelle

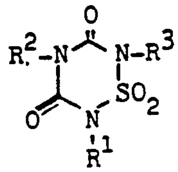

| Wirkstoff Nr. | $R^1$ | $R^2$ | $R^3$ | Fp (°C) |
|---|---|---|---|---|
| 8 | H | $CH_3$ | 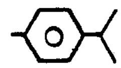 | |
| 9 | Na | $CH_3$ | " | |
| 10 | $HN(C_2H_5)_3$ | $CH_3$ | " | |
| 11 | H | $i-C_3H_7$ | " | |
| 12 | Na | $i-C_3H_7$ | " | |
| 13 | $HN(C_2H_5)_3$ | $i-C_3H_7$ | " | |
| 14 | $CH_3$ | H | " | 185 Zers. |
| 15 | $CH_3$ | Na | " | |
| 16 | $i-C_3H_7$ | H | " | 130-133 |
| 17 | $i-C_3H_7$ | Na | " | > 240 |
| 18 | $CH_3$ | $CH_3$ | " | 77-79 |
| 19 | $i-C_3H_7$ | $CH_3$ | " | 120 Zers. |
| 20 | H | $CH_3$ | 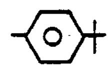 | |
| 21 | Na | $CH_3$ | " | |
| 22 | $HN(C_2H_5)_3$ | $CH_3$ | " | |
| 23 | H | $i-C_3H_7$ | " | |
| 24 | Na | $i-C_3H_7$ | " | |
| 25 | $HN(C_2H_5)_3$ | $i-C_3H_7$ | " | |
| 26 | $CH_3$ | H | " | |
| 27 | $CH_3$ | Na | " | |
| 28 | $i-C_3H_7$ | H | " | |
| 29 | $i-C_3H_7$ | Na | " | |
| 30 | $CH_3$ | $CH_3$ | " | |

(Fortsetzung)

| Wirkstoff Nr. | $R^1$ | $R^2$ | $R^3$ | Fp (°C) |
|---|---|---|---|---|
| 31 | $i-C_3H_7$ | $CH_3$ | " | |
| 32 | H | $CH_3$ | 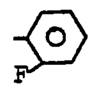 | |
| 33 | Na | $CH_3$ | " | |
| 34 | $HN(C_2H_5)_3$ | $CH_3$ | " | |
| 35 | H | $i-C_3H_7$ | " | |
| 36 | Na | $i-C_3H_7$ | " | |
| 37 | $HN(C_2H_5)_3$ | $i-C_3H_7$ | " | |
| 38 | $CH_3$ | H | " | |
| 39 | $CH_3$ | Na | " | |
| 40 | $i-C_3H_7$ | H | " | |
| 41 | $i-C_3H_7$ | Na | " | |
| 42 | $CH_3$ | $CH_3$ | " | |
| 43 | $i-C_3H_7$ | $CH_3$ | " | |
| 44 | H | $CH_3$ | 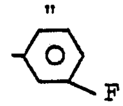 | |
| 45 | Na | $CH_3$ | " | |
| 46 | $HN(C_2H_5)_3$ | $CH_3$ | " | |
| 47 | H | $i-C_3H_7$ | " | |
| 48 | Na | $i-C_3H_7$ | " | |
| 49 | $HN(C_2H_5)_3$ | $i-C_3H_7$ | " | |
| 50 | $CH_3$ | H | " | |
| 51 | $CH_3$ | Na | " | |
| 52 | $i-C_3H_7$ | H | 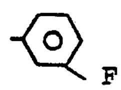 | |
| 53 | $i-C_3H_7$ | Na | " | |
| 54 | $CH_3$ | $CH_3$ | " | |
| 55 | $i-C_3H_7$ | $CH_3$ | " | |
| 56 | H | $CH_3$ | 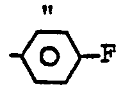 | |
| 57 | Na | $CH_3$ | " | |

(Fortsetzung)

| Wirkstoff Nr. | $R^1$ | $R^2$ | $R^3$ | Fp (°C) |
|---|---|---|---|---|
| 58 | $HN(C_2H_5)_3$ | $CH_3$ | " | |
| 59 | H | i-$C_3H_7$ | " | |
| 60 | Na | i-$C_3H_7$ | " | |
| 61 | $HN(C_2H_5)_3$ | i-$C_3H_7$ | " | |
| 62 | $CH_3$ | H | " | |
| 63 | $CH_3$ | Na | " | |
| 64 | i-$C_3H_7$ | H | " | |
| 65 | i-$C_3H_7$ | Na | " | |
| 66 | $CH_3$ | $CH_3$ | " | |
| 67 | i-$C_3H_7$ | $CH_3$ | " | |
| 68 | H | $CH_3$ | 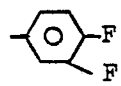 | |
| 69 | Na | $CH_3$ | " | |
| 70 | $HN(C_2H_5)_3$ | $CH_3$ | " | |
| 71 | H | i-$C_3H_7$ | " | |
| 72 | Na | i-$C_3H_7$ | " | |
| 73 | $HN(C_2H_5)_3$ | i-$C_3H_7$ | " | |
| 74 | $CH_3$ | H | " | |
| 75 | $CH_3$ | Na | " | |
| 76 | i-$C_3H_7$ | H | " | |
| 77 | i-$C_3H_7$ | Na | " | |
| 78 | $CH_3$ | $CH_3$ | " | |
| 79 | i-$C_3H_7$ | $CH_3$ | " | |
| 80 | H | $CH_3$ | 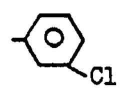 | |
| 81 | Na | $CH_3$ | " | |
| 82 | $HN(C_2H_5)_3$ | $CH_3$ | " | |
| 83 | H | i-$C_3H_7$ | " | |
| 84 | Na | i-$C_3H_7$ | " | |
| 85 | $HN(C_2H_5)_3$ | i-$C_3H_7$ | " | |
| 86 | $CH_3$ | H | " | 160 Zers. |

(Fortsetzung)

| Wirkstoff Nr. | $R^1$ | $R^2$ | $R^3$ | Fp (°C) |
|---|---|---|---|---|
| 87 | $CH_3$ | Na | " | |
| 88 | $i\text{-}C_3H_7$ | H | " | |
| 89 | $i\text{-}C_3H_7$ | Na | " | |
| 90 | $CH_3$ | $CH_3$ | " | |
| 91 | $i\text{-}C_3H_7$ | $CH_3$ | " | |
| 92 | H | $CH_3$ | 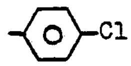 | > 240 |
| 93 | $HN(C_2H_5)_3$ | $CH_3$ | " | |
| 94 | H | $i\text{-}C_3H_7$ | " | |
| 95 | Na | $i\text{-}C_3H_7$ | " | |
| 96 | $HN(C_2H_5)_3$ | $i\text{-}C_3H_7$ | " | |
| 97 | $CH_3$ | Na | " | |
| 98 | $i\text{-}C_3H_7$ | Na | " | |
| 99 | $i\text{-}C_3H_7$ | $CH_3$ | " | |
| 100 | H | $CH_3$ | 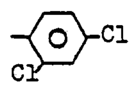 | |
| 101 | Na | $CH_3$ | " | |
| 102 | $HN(C_2H_5)_3$ | $CH_3$ | " | |
| 103 | H | $i\text{-}C_3H_7$ | " | |
| 104 | Na | $i\text{-}C_3H_7$ | " | |
| 105 | $HN(C_2H_5)_3$ | $i\text{-}C_3H_7$ | " | |
| 106 | $CH_3$ | H | " | |
| 107 | $CH_3$ | Na | " | |
| 108 | $i\text{-}C_3H_7$ | H | " | |
| 109 | $i\text{-}C_3H_7$ | Na | " | |
| 110 | $CH_3$ | $CH_3$ | " | |
| 111 | $i\text{-}C_3H_7$ | $CH_3$ | " | |
| 112 | $NH_4$ | " | 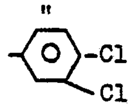 | |
| 113 | H | $C_2H_5$ | " | |

(Fortsetzung)

| Wirkstoff Nr. | $R^1$ | $R^2$ | $R^3$ | Fp (°C) |
|---|---|---|---|---|
| 114 | Na | " | " | |
| 115 | H | n-$C_3H_7$ | " | |
| 116 | Na | " | " | |
| 117 | H | i-$C_3H_7$ | " | |
| 118 | Na | " | " | |
| 119 | $NH_4$ | " | " | |
| 120 | $HN(C_2H_5)_3$ | " | " | |
| 121 | H | n-$C_4H_9$ | " | |
| 122 | Na | " | " | |
| 123 | H | i-$C_4H_9$ | " | |
| 124 | Na | " | " | |
| 125 | H | sek.-$C_4H_9$ | " | |
| 126 | Na | " | " | |
| 127 | H | t-$C_4H_9$ | " | |
| 128 | Na | " | " | |
| 129 | H | 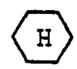 | | |
| 130 | $CH_3$ | H | " | 168-172 |
| 131 | " | Na | " | |
| 132 | $C_2H_5$ | H | " | |
| 133 | " | Na | " | |
| 134 | i-$C_3H_7$ | H | " | 120-122 |
| 135 | " | Na | " | 240 |
| 136 | $CH_3$ | $CH_3$ | " | 92 Zers. |
| 137 | i-$C_3H_7$ | $CH_3$ | " | 96-98 |
| 138 | H | $CH_3$ | 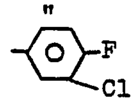 | 146-148 |
| 139 | Na | $CH_3$ | " | |
| 140 | $HN(C_2H_5)_3$ | $CH_3$ | " | |
| 141 | H | i-$C_3H_7$ | " | |
| 142 | Na | i-$C_3H_7$ | " | |

(Fortsetzung)

| Wirkstoff Nr. | $R^1$ | $R^2$ | $R^3$ | Fp (°C) |
|---|---|---|---|---|
| 143 | $HN(C_2H_5)_3$ | i-$C_3H_7$ | " | |
| 144 | $CH_3$ | H | " | |
| 145 | $CH_3$ | Na | " | |
| 146 | i-$C_3H_7$ | H | " | |
| 147 | i-$C_3H_7$ | Na | " | |
| 148 | $CH_3$ | $CH_3$ | " | |
| 149 | i-$C_3H_7$ | $CH_3$ | " | |
| 150 | H | $CH_3$ | 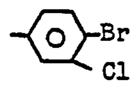 | |
| 151 | Na | $CH_3$ | " | |
| 152 | $HN(C_2H_5)_3$ | $CH_3$ | " | |
| 153 | H | i-$C_3H_7$ | " | |
| 154 | Na | i-$C_3H_7$ | " | |
| 155 | $HN(C_2H_5)_3$ | i-$C_3H_7$ | 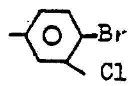 | |
| 156 | $CH_3$ | H | " | |
| 157 | $CH_3$ | Na | " | |
| 158 | i-$C_3H_7$ | H | " | |
| 159 | i-$C_3H_7$ | Na | " | |
| 160 | $CH_3$ | $CH_3$ | " | |
| 161 | i-$C_3H_7$ | $CH_3$ | " | |
| 162 | H | $CH_3$ | 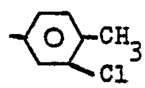 | |
| 163 | Na | $CH_3$ | " | |
| 164 | $HN(C_2H_5)_3$ | $CH_3$ | " | |
| 165 | H | i-$C_3H_7$ | " | |
| 166 | Na | i-$C_3H_7$ | " | |
| 167 | $HN(C_2H_5)_3$ | i-$C_3H_7$ | " | |
| 168 | $CH_3$ | H | " | |
| 169 | $CH_3$ | Na | " | |
| 170 | i-$C_3H_7$ | H | " | |

(Fortsetzung)

| Wirkstoff Nr. | $R^1$ | $R^2$ | $R^3$ | Fp (°C) |
|---|---|---|---|---|
| 171 | i-$C_3H_7$ | Na | " | |
| 172 | $CH_3$ | $CH_3$ | " | |
| 173 | i-$C_3H_7$ | $CH_3$ | " | |
| 174 | H | $CH_3$ | 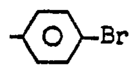 | |
| 175 | Na | $CH_3$ | " | |
| 176 | $HN(C_2H_5)_3$ | $CH_3$ | " | |
| 177 | H | i-$C_3H_7$ | " | |
| 178 | Na | i-$C_3H_7$ | " | |
| 179 | $HN(C_2H_5)_3$ | i-$C_3H_7$ | " | |
| 180 | $CH_3$ | H | " | |
| 181 | $CH_3$ | Na | " | |
| 182 | i-$C_3H_7$ | H | " | |
| 183 | i-$C_3H_7$ | Na | 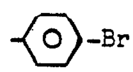 | |
| 184 | $CH_3$ | $CH_3$ | " | |
| 185 | i-$C_3H_7$ | $CH_3$ | " | |

Die erfindungsgemäßen Wirkstoffe können beispielsweise in Form von direkt versprühbaren Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen augewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali-und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergetellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile der Verbindung gemäß Beispiel 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung gemäß Beispiel 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 8 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl beteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung Nr. 8 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichsteile der Verbindung Nr. 32 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 12 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 66 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 35 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder bei Nachauflaufanwendung erfolgen. Dabei können die Mittel auf den Standort aufgebracht werden, bevor die unerwünschten Pflanzen aus den Samen gekeimt oder aus vegetativen Pflanzenteilen ausgetrieben haben, oder sie werden auf die Blätter der unerwünschten Pflanzen und der Kulturpflanzen appliziert. Vorzugsweise werden die neuen Wirkstoffe nach dem Auflaufen der unerwünschten Pflanzen, sowohl auf Kulturflächen als auch auf unbebautem Land, ausgebracht. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit und Wachstumsstadium 0,1 bis 15 kg/ha und mehr, wobei sich die höheren Dosen besonders zur totalen Bekämpfung von Vegetationen eignen.

Der Einfluß verschiedener Vertreter der erfindungsgemäßen Verbindungen auf das Wachstum von unerwünschten Pflanzen im Vergleich zu bekannten Wirkstoffen wird durch Gewächshausversuche gezeigt :

Als Kulturgefäße dienten 300 $cm^3$ große Plastikblumentöpfe mit lehmigem Sand und etwa 1,5 % Humus als Substrat. Die Samen der in der nachfolgenden Tabelle 1 aufgeführten Testpflanzen wurden nach Arten getrennt flach eingesät.

(Siehe Tabelle 1 Seite 18 f.)

Tabelle 1 — Liste der Pflanzennamen

| Botanischer Name | Abkürzung in Tabelle | Deutscher Name | Englischer Name |
|---|---|---|---|
| Abutilon theophrasti | Abutilon theophr. | Chinesischer Hanf | velvet leaf |
| Beta vulgaris | - | Zuckerrübe | Sugarbeet |
| Chenopodium album | - | Weißer Gänsefuß | lambsquarters |
| Chrysanthemum segetum | Chrysanthemum seg. | Saatwucherblume | corn marigold |
| Centaurea cyanus | Centaurea cyan. | Kornblume | cornflower |
| Gossypium hirsutum | - | Baumwolle | cotton |
| Matricaria spp. | Matric. spp. | Kamillearten | chamomile |
| Nicandra physaloides | Nicandra physal. | Giftbeere | apple-of-Peru |
| Sinapis alba | - | Weißer Senf | white mustard |
| Solanum nigrum | Solanum nigr. | Schwarzer Nacht-schatten | black nightshade |
| Sorghum bicolor | - | Mohrenhirse (Kulturhirse) | sorghum |
| Triticum aestivum | - | Weizen | wheat |
| Zea mays | - | Mais | Indian corn |

Unmittelbar danach erfolgte bei Vorauflaufbehandlung das Aufbringen des Wirkstoffes auf die Erdoberfläche. Sie wurden hierbei in Wasser als Verteilungsmittel suspendiert oder emulgiert und mittels fein verteilender Düsen gespritzt. Dabei handelte es sich bei dieser Applikationsmethode um eine Aufwandmenge von 2,0 kg/ha Wirkstoff.

Nach dem Aufbringen des Mittels wurden die Gefäße leicht beregnet, um Keimung und Wachstum in Gang zu bringen und die chemischen Mittel zu aktivieren. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Chemikalien beeinträchtigt wurde. Zum Zwecke der Nachauflaufbehandlung zog man die Testpflanzen je nach Wuchsform erst bis zu einer Wuchshöhe von 3 bis 10 cm an und behandelte sie dann. Zur Nachauflaufbehandlung wurden entweder direkt gesäte und in den gleichen Gefäßen aufgewachsene Pflanzen ausgewählt, oder sie wurden erst als Keimpflanzen separat angezogen und einige Tage vor der Behandlung in die Versuchsgefäße transplantiert. Die Aufwandmengen für die Nachauflaufbehandlung war 1,0 kg/ha Wirkstoff.

Die Abdeckung unterblieb bei der Nachauflaufbehandlung. Die Aufstellung der Versuche erfolgte im Gewächshaus, wobei für wärmeliebende Arten wärmere Bereiche (20 bis 30 °C) und für solche gemäßigter Klimate 15 bis 30 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Auflauf und 100 kein Aufgang der Pflanzen bzw. völlige Zerstörung zumindest der oberirdischen Sproßteile.

Als Vergleichssubstanz diente 2,4-Diisopropyl-3,4,5,6-tetrahydro-1,2,4,6-thiatriazin(3,5)dion-1,1-dioxid (I) (vgl. US-PS 3 435 031). Die Aufwandmenge von I betrug 1,0 kg Wirkstoff/ha.

Bei diesen Versuchen zeigte beispielsweise der Wirkstoff des Beispiels Nr. 138 bei 2 kg/ha Vorauflaufanwendung eine beachtliche herbizide Aktivität.

Die Gewächshausversuche ergaben ferner bei Nachauflaufanwendung z. B. des Wirkstoffes des Beispiels 4 mit 1,0 kg/ha eine dem Vergleichsmittel I überlegene selektive herbizide Wirkung. Ähnliches gilt für die Substanz des Beispiels 18.

Es ist nützlich, die erfindungsgemäßen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Zur Aktivierung der herbiziden Wirkung können auch Netz- und Haftmittel sowie nicht-phytotoxische Öle und Ölkonzentrate zugesetzt werden.

**Ansprüche** (für die Vertragsstaaten : BE, CH, DE, FR, GB, IT, LI, NL, SE)

1. 3,4,5,6-Tetrahydro-thiatriazin(3,5)dion-1,1-dioxide der Formel I

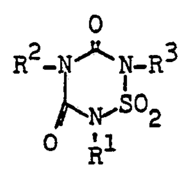

(I)

worin

$R^1$ Wasserstoff, ein Metallatom, einen gegebenenfalls $C_{1-3}$-alkylsubstituierten Ammoniumrest, einen gesättigten geradkettigen aliphatischen Rest mit bis zu 10 Kohlenstoffatomen, einen cycloaliphatischen Rest mit 3 bis 7 Kohlenstoffatomen, einen verzweigten gesättigten aliphatischen Rest mit 3 bis 10 Kohlenstoffatomen, oder einen gegebenenfalls halogensubstituierten Benzylrest,

$R^2$ Wasserstoff, ein Metallatom, einen gesättigten aliphatischen Rest mit bis zu 10 Kohlenstoffatomen,

$R^3$ einen halogen-, alkyl-, halogenalkylthio-, halogenalkylsulfonyl-substituierten Phenylrest mit bis zu 10 Kohlenstoffatomen bedeuten.

2. 3,4,5,6-Tetrahydro-thiatriazin(3,5)dion-1,1-dioxide der Formel I gemäß Anspruch 1, dadurch gekennzeichnet, daß $R^1$ für Wasserstoff, $R^2$ für Alkyl mit 1 bis 4 Kohlenstoffatomen und $R^3$ für einen halogensubstituierten Phenylrest stehen.

3. Herbizid, dadurch gekennzeichnet, daß es ein Tetrahydro-thiatriazin(3,5)dion-1,1-dioxid der Formel I gemäß Anspruch 1 enthält.

4. Verfahren zur Bekämpfung unerwünschten Pflanzenwuchses, dadurch gekennzeichnet, daß man die Pflanzen oder den Boden behandelt mit einem Tetrahydro-thiatriazin(3,5)dion-1,1-dioxid der Formel I gemäß Anspruch 1.

5. Verfahren zur Herstellung von Tetrahydro-thiatriazin(3,5)dion-1,1-dioxiden gemäß Anspruch 1, dadurch gekennzeichnet, daß man

a) — falls $R^1$ Wasserstoff bedeutet — einen N-Aryl-N'-Alkyl-Harnstoff der Formel II

$$R^2\text{-NH-}\overset{\overset{\displaystyle O}{\|}}{C}\text{-NH-}R^3 \qquad \text{(II)}$$

in der $R^2$ und $R^3$ die vorgenannten Bedeutungen besitzen, mit Chlorsulfonylisocyanat, gegebenenfalls in Gegenwart eines Säureakzeptors und eines inerten Lösungsmittels bei einer Temperatur im Bereich von − 40 bis 100 °C oder

b) — falls $R^2$ Wasserstoff bedeutet — ein Sulfondiamid der Formel III

$$R^1\text{—NH—}SO_2\text{—NH—}R^3 \qquad \text{(III)}$$

worin $R^1$ und $R^3$ die vorgenannten Bedeutungen besitzen, mit Chlorcarbonylisocyanat, gegebenenfalls in Gegenwart eines Säureakzeptors und eines inerten organischen Lösungsmittels bei einer Temperatur im Bereich von 0 °C bis 150 °C oder

c) einen Harnstoff der Formel II

$$R^2\text{-NH-}\overset{\overset{\displaystyle O}{\|}}{C}\text{-NH-}R^3 \qquad \text{(II)}$$

oder einen Harnstoff der Formel IV

$$R^2\text{-NH-}\overset{\overset{\displaystyle O}{\|}}{C}\text{-}NH_2 \qquad \text{(IV)}$$

worin $R^2$ und $R^3$ die vorgenannte Bedeutung besitzen, mit einem Sulfamoylchlorid der Formel V

$$R^1\text{-}\underset{\underset{\displaystyle COCl}{|}}{N}\text{-}SO_2Cl \qquad \text{(V)}$$

oder einem Sulfamoylchlorid der Formel VI

$$R^2\text{-}\underset{\underset{\displaystyle CO_2R^4}{|}}{N}\text{-}SO_2\text{-}Cl \qquad \text{(VI)}$$

in der $R^1$ die vorgenannte Bedeutung, ausgenommen Wasserstoff, ein Metallatom oder einen gegebenenfalls $C_{1-3}$-alkylsubstituierten Ammoniumrest besitzt, und $R^4$ Alkyl oder Cycloalkyl mit bis zu 6 Kohlenstoffatomen bedeutet, gegebenenfalls in Gegenwart eines Säureakzeptors und eines inerten Lösungsmittels bei einer Temperatur von − 20 bis 100 °C oder

d) ein Sulfondiamid der Formel III mit einem Carbamoylchlorid der Formel VII

$$R^2\text{-}\underset{\underset{\displaystyle CO\text{-}Cl}{|}}{N}\text{-CO-O}R^4 \qquad \text{(VII)}$$

oder einem Carbamoylchlorid der Formel VIII

$$R^2\text{-}\underset{\underset{\displaystyle CO\text{-}Cl}{|}}{N}\text{-CO-Cl} \qquad \text{(VIII)}$$

in der $R^2$ und $R^4$ die vorgenannte Bedeutung, ausgenommen Wasserstoff oder Metallatome besitzen, gegebenenfalls in Gegenwart eines Säureakzeptors und eines inerten Lösungsmittels bei einer Temperatur von – 20 °C bis 100 °C oder

e) — falls $R^2$ Wasserstoff bedeutet — ein Sulfamoylchlorid der Formel IX

$$R^1-N-SO_2Cl \qquad (IX)$$
$$CO-NCO$$

in der $R^1$ die vorgenannte Bedeutung besitzt, ausgenommen Wasserstoff, ein Metallatom und einen gegebenenfalls $C_{1-3}$-alkylsubstituierten Ammoniumrest, mit einem Amin der Formel X

$$R^3—NH_2 \qquad (X)$$

in der $R^3$ die vorgenannte Bedeutung besitzt, gegebenenfalls in Gegenwart eines Säureakzeptors und eines inerten Lösungsmittels bei einer Temperatur von – 100 °C bis 100 °C reagieren läßt und die so erhaltenen Verbindungen gegebenenfalls in ihre Salze überführt und/oder — falls $R^1$ Wasserstoff bedeutet — alkyliert.

6. Verfahren zur Herstellung von Herbiziden, dadurch gekennzeichnet, daß man mindestens eine Verbindung der Formel I gemäß Anspruch 1 mit festen oder flüssigen Trägerstoffen mischt.

**Ansprüche** (für den Vertragsstaat AT)

1. Verfahren zur Herstellung von Tetrahydro-thiatriazin(3,5)dion-1,1-dioxiden, dadurch gekennzeichnet, daß man 3,4,5,6-Tetrahydro-thiatriazin(3,5)dion-1,1-dioxide der Formel I

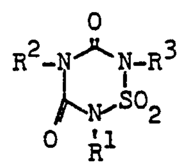

(I)

worin

$R^1$ Wasserstoff, ein Metallatom, einen gegebenenfalls $C_{1-3}$-alkylsubstituierten Ammoniumrest, einen gesättigten geradkettigen aliphatischen Rest mit bis zu 10 Kohlenstoffatomen, einen cycloaliphatischen Rest mit 3 bis 7 Kohlenstoffatomen, einen verzweigten gesättigten aliphatischen Rest mit 3 bis 10 Kohlenstoffatomen, oder einen gegebenenfalls halogensubstituierten Benzylrest,

$R^2$ Wasserstoff, ein Metallatom, einen gesättigten aliphatischen Rest mit bis zu 10 Kohlenstoffatomen,

$R^3$ einen halogen-, alkyl-, halogenalkylthio-, halogenalkylsulfonyl-substituierten Phenylrest mit bis zu 10 Kohlenstoffatomen bedeuten, herstellt, indem man

a) — falls $R^1$ Wasserstoff bedeutet — einen N-Aryl-N'-Alkyl-Harnstoff der Formel II

$$R^2-NH-\overset{O}{\overset{\|}{C}}-NH-R^3 \qquad (II)$$

in der $R^2$ und $R^3$ die vorgenannten Bedeutungen besitzen, mit Chlorsulfonylisocyanat, gegebenenfalls in Gegenwart eines Säureakzeptors und eines inerten Lösungsmittels bei einer Temperatur im Bereich von – 40 bis 100 °C oder

b) — falls $R^2$ Wasserstoff bedeutet — ein Sulfondiamid der Formel III

$$R^1—NH—SO_2—NH—R^3 \qquad (III)$$

worin $R^1$ und $R^3$ die vorgenannten Bedeutungen besitzen, mit Chlorcarbonylisocyanat, gegebenenfalls in Gegenwart eines Säureakzeptors und eines inerten organischen Lösungsmittels bei einer Temperatur im Bereich von 0 °C bis 150 °C oder

c) einen Harnstoff der Formel II

$$R^2-NH-\overset{O}{\overset{\|}{C}}-NH-R^3 \qquad (II)$$

oder einen Harnstoff der Formel IV

$$R^2-NH-\overset{O}{\overset{\|}{C}}-NH_2 \qquad (IV)$$

worin $R^2$ und $R^3$ die vorgenannte Bedeutung besitzen, mit einem Sulfamoylchlorid der Formel V

$$\begin{array}{l} R^1-N-SO_2Cl \\ \quad\;\; | \\ \quad\;\; COCl \end{array} \qquad (V)$$

oder einem Sulfamoylchlorid der Formel VI

$$\begin{array}{l} R^2-N-SO_2-Cl \\ \quad\;\; | \\ \quad\;\; CO_2R^4 \end{array} \qquad (VI)$$

in der $R^1$ die vorgenannte Bedeutung, ausgenommen wasserstoff, ein Metallatom oder einen gegebenenfalls $C_{1-3}$-alkylsubstituierten Ammoniumrest besitzt, und $R^4$ Alkyl oder Cycloalkyl mit bis zu 6 Kohlenstoffatomen bedeutet, gegebenenfalls in Gegenwart eines Säureakzeptors und eines inerten Lösungsmittels bei einer Temperatur von − 20 bis 100 °C oder

d) ein Sulfondiamid der Formel III mit einem Carbamoylchlorid der Formel VII

$$\begin{array}{l} R^2-N-CO-OR^4 \\ \quad\;\; | \\ \quad\;\; CO-Cl \end{array} \qquad (VII)$$

oder einem Carbamoylchlorid der Formel VIII

$$\begin{array}{l} R^2-N-CO-Cl \\ \quad\;\; | \\ \quad\;\; CO-Cl \end{array} \qquad (VIII)$$

in der $R^2$ und $R^4$ die vorgenannte Bedeutung, ausgenommen Wasserstoff oder Metallatome besitzen, gegebenenfalls in Gegenwart eines Säureakzeptors und eines inerten Lösungsmittels bei einer Temperatur von − 20 °C bis 100 °C oder

e) — falls $R^2$ Wasserstoff bedeutet — ein Sulfamoylchlorid der Formel IX

$$\begin{array}{l} R^1-N-SO_2Cl \\ \quad\;\; | \\ \quad\;\; CO-NCO \end{array} \qquad (IX)$$

in der $R^1$ die vorgenannte Bedeutung besitzt, ausgenommen Wasserstoff, ein Metallatom und einen gegebenenfalls $C_{1-3}$-alkylsubstituierten Ammoniumrest, mit einem Amin der Formel X

$$R^3—NH_2 \qquad (X)$$

in der $R^3$ die vorgenannte Bedeutung besitzt, gegebenenfalls in Gegenwart eines Säureakzeptors und eines inerten Lösungsmittels bei einer Temperatur von − 100 °C bis 100 °C
reagieren läßt und die so erhaltenen Verbindungen gegebenenfalls in ihre Salze überführt und/oder — falls $R^1$ Wasserstoff bedeutet — alkyliert.

2. Herbizid, dadurch gekennzeichnet, daß es ein Tetrahydro-thiatriazin(3,5)dion-1,1-dioxid der Formel I gemäß Anspruch 1 enthält.

3. Verfahren zur Bekämpfung unerwünschten Pflanzenwuchses, dadurch gekennzeichnet, daß man die Pflanzen oder den Boden behandelt mit einem Tetrahydro-thiatriazin(3,5)dion-1,1-dioxid der Formel I gemäß Anspruch 1.

**Claims** (for the Contracting States : BE, CH, DE, FR, GB, IT, LI, NL, SE)

1. A 3,4,5,6-tetrahydro-thiatriazine-3,5-dione-1,1-dioxide of the formula I

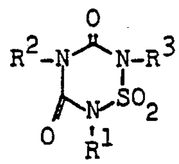

(I)

where

$R^1$ is hydrogen, a metal atom, an unsubstituted or $C_1$-$C_3$-alkyl-substituted ammonium radical, a saturated straight-chain aliphatic radical of up to 10 carbon atoms, a cycloaliphatic radical of 3 to 7 carbon atoms, a branched saturated aliphatic radical of 3 to 10 carbon atoms or an unsubstituted or halogen-substituted benzyl radical,

$R^2$ is hydrogen, a metal atom or a saturated aliphatic radical of up to 10 carbon atoms, and

$R^3$ is a halogen-, alkyl-, haloalkylthio- or haloalkylsulfonyl-substituted phenyl radical of up to 10 carbon atoms.

2. A 3,4,5,6-tetrahydro-thiatriazine-3,5-dione-1,1-dioxide of the formula I as claimed in claim 1, where $R^1$ denotes hydrogen, $R^2$ denotes alkyl of 1 to 4 carbon atoms, and $R^3$ denotes halogen-substituted phenyl.

3. A herbicide containing a tetrahydrothiatriazine-3,5-dione-1,1-dioxide of the formula I as claimed in claim 1.

4. A process for combating the growth of unwanted plants, wherein the plants or the soil are treated with a tetrahydro-thiatriazine-3,5-dione-1,1 dioxide of the formula I as claimed in claim 1.

5. A process for the preparation of a tetrahydro-thiatriazine-3,5-dione-1,1-dioxide as claimed in claim 1, wherein

a) if $R^1$ is hydrogen, an N-aryl-N'-alkyl-urea of the formula II

$$R^2-NH-\overset{O}{\overset{\|}{C}}-NH-R^3 \qquad (II)$$

where $R^2$ and $R^3$ have the above meanings, is reacted with chlorosulfonyl isocyanate, in the presence or absence of an acid acceptor and of an inert solvent, at from −40 to 100 °C, or

b) if $R^2$ is hydrogen, a sulfonediamide of the formula III

$$R^1—NH—SO_2—NH—R^3 \qquad (III)$$

where $R^1$ and $R^3$ have the above meanings, is reacted with chlorocarbonyl isocyanate, in the presence or absence of an acid acceptor and of an inert organic solvent, at from 0° to 150 °C, or

c) a urea of the formula II

$$R^2-NH-\overset{O}{\overset{\|}{C}}-NH-R^3 \qquad (II)$$

or a urea of the formula IV

$$R^2—NH—CO—NH_2 \qquad (IV)$$

where $R^2$ and $R^3$ have the above meanings, is reacted with a sulfamyl chloride of the formula V

$$\begin{array}{l} R^1-N-SO_2Cl \\ \quad\;\, | \\ \quad\;\, COCl \end{array} \qquad (V)$$

or a sulfamyl chloride of the formula VI

$$\begin{array}{l} R^1\text{-}N\text{-}SO_2\text{-}Cl \\ \quad\;\; | \\ \quad\; CO_2R^4 \end{array} \qquad (VI)$$

where $R^1$ has the above meanings, with the exception of hydrogen, a metal atom and an unsubstituted or $C_1$-$C_3$-alkyl-substituted ammonium radical, and $R^4$ is alkyl or cycloalkyl of up to 6 carbon atoms, in the presence or absence of an acid acceptor and of an inert solvent, at from − 20 to 100 °C, or

d) a sulfonediamide of the formula III is reacted with a carbamyl chloride of the formula VII

$$\begin{array}{l} R^2\text{-}N\text{-}CO\text{-}OR^4 \\ \quad\;\; | \\ \quad\; CO\text{-}Cl \end{array} \qquad (VII)$$

or a carbamyl chloride of the formula VIII

$$\begin{array}{l} R^2\text{-}N\text{-}CO\text{-}Cl \\ \quad\;\; | \\ \quad\; CO\text{-}Cl \end{array} \qquad (VIII)$$

where $R^2$ and $R^4$ have the above meanings, with the exception of hydrogen and a metal atom, in the presence or absence of an acid acceptor and of an inert solvent, at from − 20 °C to 100 °C, or

e) if $R^2$ is hydrogen, a sulfamyl chloride of the formula IX

$$\begin{array}{l} R^1\text{-}N\text{-}SO_2Cl \\ \quad\;\; | \\ \quad\; CO\text{-}NCO \end{array} \qquad (IX)$$

where $R^1$ has the above meanings, with the exception of hydrogen, a metal atom and an unsubstituted or $C_1$-$C_3$-alkyl-substituted ammonium radical, is reacted with an amine of the formula X

$$R^3—NH_2 \qquad (X)$$

where $R^3$ has the above meanings, in the presence or absence of an acid acceptor and of an inert solvent, at from − 100 °C to 100 °C, and, in each case, the compound obtained may, if desired, be converted into a salt and/or, if $R^1$ is hydrogen, alkylated.

6. A process for the manufacture of herbicides, wherein at least one compound of the formula I as claimed in claim 1 is mixed with a solid or liquid carrier.

**Claims** (for the Contracting State AT)

1. A process for the preparation of a tetrahydro-thiatriazine-3,5-dione-1,1-dioxide, wherein a 3,4,5,6-tetrahydro-thiatriazine-3,5-dione-1,1-dioxide of the formula I

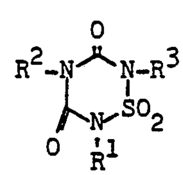

(I)

where

$R^1$ is hydrogen, a metal atom, an unsubstituted or $C_1$-$C_3$-alkyl-substituted ammonium radical, a saturated straight-chain aliphatic radical of up to 10 carbon atoms, a cycloaliphatic radical of 3 to 7 carbon atoms, a branched saturated aliphatic radical of 3 to 10 carbon atoms or an unsubstituted or halogen-substituted benzyl radical,

$R^2$ is hydrogen, a metal atom or a saturated aliphatic radical of up to 10 carbon atoms, and

$R^3$ is a halogen-, alkyl-, haloalkylthio- or haloalkylsulfonyl-substituted phenyl radical of up to 10 carbon atoms, is prepared wherein

a) if $R^1$ is hydrogen, by reaching an N-aryl-N'-alkyl-urea of the formula II

$$R^2\text{-NH-}\overset{\overset{O}{\|}}{C}\text{-NH-}R^3 \qquad (II)$$

where $R^2$ and $R^3$ have the above meanings, with chlorosulfonyl isocyanate, in the presence or absence of an acid acceptor and of an inert solvent, at from −40 to 150 °C, or

b) if $R^2$ is hydrogen, by reaching a sulfonediamide of the formula III

$$R^1\text{—NH—}SO_2\text{—NH—}R^3 \qquad (III)$$

where $R^1$ and $R^3$ have the above meanings, with chlorocarbonyl isocyanate, in the presence or absence of an acid acceptor and of an inert organic solvent, at from 0° to 150 °C, or

c) by reaching a urea of the formula II

$$R^2\text{-NH-}\overset{\overset{O}{\|}}{C}\text{-NH-}R^3 \qquad (II)$$

or a urea of the formula IV

$$R^2\text{—NH—CO—}NH_2 \qquad (IV)$$

where $R^2$ and $R^3$ have the above meanings, with a sulfamylchloride of the formula V

$$R^1\text{-}\underset{\underset{COCl}{|}}{N}\text{-}SO_2Cl \qquad (V)$$

or a sulfamyl chloride of the formula VI

$$R^1\text{-}\underset{\underset{CO_2R^4}{|}}{N}\text{-}SO_2\text{-}Cl \qquad (VI)$$

where $R^1$ has the above meanings, with the exception of hydrogen, a metal atom and an unsubstituted or $C_1$-$C_3$-alkyl-substituted ammonium radical, and $R^4$ is alkyl or cycloalkyl of up to 6 carbon atoms, in the presence or absence of an acid acceptor and of an inert solvent, at from −20 to 100 °C, or

d) by reaching a sulfonediamide of the formula III with a carbamyl chloride of the formula VII

$$R^2\text{-}\underset{\underset{CO\text{-}Cl}{|}}{N}\text{-CO-O}R^4 \qquad (VII)$$

or a carbamyl chloride of the formula VIII

$$R^2\text{-}\underset{\underset{CO\text{-}Cl}{|}}{N}\text{-CO-Cl} \qquad (VIII)$$

where $R^2$ and $R^4$ have the above meanings, with the exception of hydrogen and a metal atom, in the presence or absence of an acid acceptor and of an inert solvent, at from −20 °C to 100 °C, or

e) if $R^2$ is hydrogen, by reaching a sulfamyl chloride of the formula IX

$$R^1\text{-}\underset{\underset{CO\text{-}NCO}{|}}{N}\text{-}SO_2Cl \qquad (IX)$$

where $R^1$ has the above meanings, with the exception of hydrogen, a metal atom and an unsubstituted or $C_1$-$C_3$-alkyl-substituted ammonium radical, with an amine of the formula X

$$R^3—NH_2 \qquad (X)$$

where $R^3$ has the above meanings, in the presence or absence of an acid acceptor and of an inert solvent, at from − 100 °C to 100 °C, and, in each case, the compound obtained may, if desired, be converted into a salt and/or, if $R^1$ is hydrogen, alkylated.

2. A herbicide containing a tetrahydrothiatriazine-3,5-dione-1,1-dioxide of the formula I as defined in claim 1.

3. A process for combating the growth of unwanted plants, wherein the plants or the soil are treated with a tetrahydro-thiatriazine-3,5-dione-1,1-dioxide of the formula I as defined in claim 1.

**Revendications** (pour les Etats contractants : BE, CH, DE, FR, GB, IT, LI, NL, SE)

1. 3,4,5,6-tetrahydro-thiatriazin(3,5)dione-1,1-dioxyde de formule I

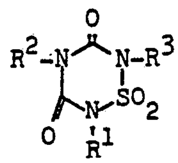

(I)

dans laquelle

$R^1$ représente hydrogène, un atome de métal, un reste d'ammonium substitué éventuellement par un alkyle en $C_{1\text{-}3}$, un reste aliphatique à chaîne droite, saturé ayant jusqu'à 10 atomes de carbone, un reste cycloaliphatique ayant 3 à 7 atomes de carbone, un reste aliphatique saturé ramifié ayant 3 à 10 atomes de carbone ou un reste benzyle éventuellement substitué par un halogène,

$R^2$ représente hydrogène, un atome de métal, un reste aliphatique saturé ayant jusqu'à 10 atomes de carbone,

$R^3$ un reste phényle, substitué par un halogène, alkyle, halogénoalkylthio, halogénoalkylsulfonyle, ayant jusqu'à 10 atomes de carbone.

2. 3,4,5,6-tetrahydro-thiatriazin(3,5)dione-1,1-dioxyde de formule I selon la revendication 1 caractérisé par le fait que $R^1$ représente hydrogène, $R^2$ représente alkyle de 1 à 4 atomes de carbone et $R^3$ un reste phényle substitué par un halogène.

3. Herbicide caractérisé par le fait qu'il contient un tetrahydro-thiatriazin(3,5)dione-1,1-dioxyde de formule I selon la revendication 1.

4. Procédé pour lutter contre la croissance indésirable des plantes, caractérisé par le fait que l'on traite les plantes ou le sol avec un tetrahydro-thiatriazin(3,5)dione-1,1-dioxyde de formule I selon la revendication 1.

5. Procédé pour la préparation de tetrahydro-thiatriazin(3,5)dione-1,1-dioxydes selon la revendication 1, caractérisé par le fait que

a) dans le cas où $R^1$ représente l'hydrogène, on fait réagir une N-aryl-N'-alkyl-urée de formule II

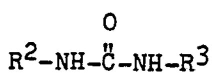

(II)

dans laquelle $R^2$ et $R^3$ ont les significations susmentionnées, avec un chlorosulfonylisocyanate, éventuellement en présence d'un accepteur d'acide et d'un solvant inerte, à une température comprise dans la zone de − 40 à 100 °C ou

b) dans le cas où $R^2$ représente hydrogène, on fait réagir un sulfodiamide de formule III

$$R^1—NH—SO_2—NH—R^3 \qquad (III)$$

dans laquelle $R^1$ et $R^3$ ont les significations susindiquées, avec un isocyanate de chlorocarbonyle, éventuellement en présence d'un accepteur d'acide et d'un solvant organique inerte, à une température située dans les limites de 0 °C à 150 °C ou

c) on fait réagir une urée de formule II

$$R^2-NH-\overset{O}{\overset{\|}{C}}-NH-R^3 \qquad (II)$$

ou une urée de formule IV

$$R^2-NH-\overset{O}{\overset{\|}{C}}-NH_2 \qquad (IV)$$

dans laquelle $R^2$ et $R^3$ ont les significations susindiquées, avec un sulfamoylchlorure de formule V

$$\begin{array}{l} R^1-N-SO_2Cl \\ \quad\;\; | \\ \quad\;\; COCl \end{array} \qquad (V)$$

ou un chlorure de sulfamyle de formule VI

$$\begin{array}{l} R^2-N-SO_2-Cl \\ \quad\;\; | \\ \quad\;\; CO_2R^4 \end{array} \qquad (VI)$$

dans laquelle $R^1$ a la signification susmentionnée, à l'exclusion de l'hydrogène, d'un atome de métal ou d'un reste d'ammonium, substitué par un alkyle en $C_{1-3}$ éventuellement, et $R^4$ représente un alkyle ou cycloalkyle ayant jusqu'à 6 atomes de carbone, éventuellement en présence d'un accepteur d'acide et d'un solvant inerte à une température de − 20 à 100 °C ou

d) on fait réagir un sulfodiamide de formule III avec un chlorure de carbamoyle de formule VII

$$\begin{array}{l} R^2-N-CO-OR^4 \\ \quad\;\; | \\ \quad\;\; CO-Cl \end{array} \qquad (VII)$$

ou un chlorure de carbamoyle de formule VIII

$$\begin{array}{l} R^2-N-CO-Cl \\ \quad\;\; | \\ \quad\;\; CO-Cl \end{array} \qquad (VIII)$$

dans laquelle $R^2$ et $R^4$ ont les significations susmentionnées, à l'exclusion de l'hydrogène ou d'un atome de métal, éventuellement en présence d'un accepteur d'acide et d'un solvant inerte, à une température de − 20 °C à 100 °C ou

e) dans le cas où $R^2$ représente l'hydrogène, on fait réagir un chlorure de sulfamyle de formule IX

$$\begin{array}{l} R^1-N-SO_2Cl \\ \quad\;\; | \\ \quad\;\; CO-NCO \end{array} \qquad (IX)$$

dans laquelle $R^1$ a la signification susmentionnée, à l'exclusion de l'hydrogène, d'un atome de métal et d'un reste d'ammonium, éventuellement substitué par un alkyle en $C_{1-3}$, avec une amine de formule X

$$R^3—NH_2 \qquad (X)$$

dans laquelle $R^3$ a la signification susmentionnée, éventuellement en présence d'un accepteur d'acide et d'un solvant inerte à une température de − 100 °C à 100 °C
et les composés ainsi obtenus sont éventuellement transformés en leurs seuls et/ou alkylés dans le cas où $R^1$ représente l'hydrogène.

6. Procédé pour la préparation d'herbicides, caractérisé par le fait que l'on mélange un composé de formule I selon la revendication 1 avec une matière support solide ou liquide.

**Revendications** (pour l'Etat contractant AT)

1. Procédé pour la préparation de tetrahydro-thiatriazin(3,5)dione-1,1-dioxydes, caractérisé par le fait qu'on prépare du 3,4,5,6-tetrahydro-thiatriazin(3,5)dione-1,1-dioxyde de formule I

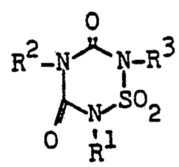

(I)

dans laquelle

$R^1$ représente hydrogène, un atome de métal, un reste d'ammonium substitué éventuellement par un alkyle en $C_{1-3}$, un reste aliphatique à chaîne droite, saturé ayant jusqu'à 10 atomes de carbone, un reste cycloaliphatique ayant 3 à 7 atomes de carbone, un reste aliphatique saturé ramifié ayant 3 à 10 atomes de carbone ou un reste benzyle éventuellement substitué par un halogène,

$R^2$ représente hydrogène, un atome de métal, un reste aliphatique saturé ayant jusqu'à 10 atomes de carbone,

$R^3$ un reste phényle, substitué par un halogène, alkyle, halogénoalkylthio, halogénoalkylsulfonyle, ayant jusqu'à 10 atomes de carbone et pour cela

a) dans le cas où $R^1$ représente l'hydrogène, on fait réagir une N-aryl-N'-alkyl-urée de formule II

$$R^2-NH-\overset{\overset{O}{\|}}{C}-NH-R^3 \qquad (II)$$

dans laquelle $R^2$ et $R^3$ ont les significations susmentionnées, avec un chlorosulfonylisocyanate, éventuellement en présence d'un accepteur d'acide et d'un solvant inerte, à une température comprise dans la zone de − 40 à 100 °C ou

b) dans le cas où $R^2$ représente hydrogène, on fait réagir un sulfodiamide de formule III

$$R^1—NH—SO_2—NH—R^3 \qquad (III)$$

$$\begin{array}{l} R^2-N-CO-OR^4 \\ \quad\ \ | \\ \quad CO-Cl \end{array} \qquad (VII)$$

ou un chlorure de carbamoyle de formule VIII

$$\begin{array}{l} R^2-N-CO-Cl \\ \quad\ \ | \\ \quad CO-Cl \end{array} \qquad (VIII)$$

dans laquelle $R^2$ et $R^4$ ont les significations susmentionnées, à l'exclusion de l'hydrogène ou d'un atome de métal, éventuellement en présence d'un accepteur d'acide et d'un solvant inerte, à une température de − 20 °C à 100 °C ou

e) dans le cas où $R^2$ représente l'hydrogène, on fait réagir un chlorure de sulfamyle de formule IX

$$\begin{array}{l} R^1-N-SO_2Cl \\ \quad\ \ | \\ \quad CO-NCO \end{array} \qquad (IX)$$

dans laquelle $R^1$ a la signification susmentionnée, à l'exclusion de l'hydrogène, d'un atome de métal et d'un reste d'ammonium, éventuellement substitué par un alkyle en $C_{1-3}$, avec une amine de formule X

$$R^3—NH_2 \qquad (X)$$

dans laquelle $R^3$ a la signification susmentionnée, éventuellement en présence d'un accepteur d'acide et d'un solvant inerte à une température de − 100 °C à 100 °C

et les composés ainsi obtenus sont éventuellement transformés en leurs sels et/ou alkylés, dans le cas où $R^1$ représente l'hydrogène.

dans laquelle $R^1$ et $R^3$ ont les significations susindiquées, avec un isocyanate de chlorocarbonyle, éventuellement en présence d'un accepteur d'acide et d'un solvant organique inerte, à une température située dans les limites de 0 °C à 150 °C ou

c) on fait réagir une urée de formule II

$$R^2-NH-\overset{O}{\overset{\|}{C}}-NH-R^3 \quad (II)$$

ou une urée de formule IV

$$R^2-NH-\overset{O}{\overset{\|}{C}}-NH_2 \quad (IV)$$

dans laquelle $R^2$ et $R^3$ ont les significations susindiquées, avec un sulfamoylchlorure de formule V

$$R^1-\underset{COCl}{\underset{|}{N}}-SO_2Cl \quad (V)$$

ou un chlorure de sulfamoyle de formule VI

$$R^2-\underset{CO_2R^4}{\underset{|}{N}}-SO_2-Cl \quad (VI)$$

dans laquelle $R^1$ a la signification susmentionnée, à l'exclusion de l'hydrogène, d'un atome de métal ou d'un reste d'ammonium, substitué par un alkyle en $C_{1-3}$ éventuellement, et $R^4$ représente un alkyle ou cycloalkyle ayant jusqu'à 6 atomes de carbone, éventuellement en présence d'un accepteur d'acide et d'un solvant inerte à une température de − 20 à 100 °C ou

d) on fait réagir un sulfodiamide de formule III avec un chlorure de carbamoyle de formule VII.

2. Herbicide caractérisé par le fait qu'il contient un tetrahydro-thiatriazin(3,5)dione-1,1-dioxyde de formule I selon la revendication 1.

3. Procédé pour lutter contre la croissance indésirable des plantes, caractérisé par le fait que l'on traite les plantes ou le sol avec un tetrahydro-thiatriazin(3,5)dione-1,1-dioxyde de formule I selon la revendication 1.